# EUROPEAN PATENT SPECIFICATION

(11) **EP 3 253 314 B1**
(45) Date of publication and mention of the grant of the patent: **19.01.2022**
(21) Application number: 15880741.2
(22) Date of filing: 06.02.2015
(51) Int. Cl.: A61B 18/12, A61B 18/14

(54) **CONTROLLING JAW FORCES WITH SPRING ASSEMBLY**
STEUERUNG VON BACKENKRÄFTEN MIT FEDERBAUGRUPPE
CONTRÔLE DES FORCES DE LA MÂCHOIRE AVEC UN ENSEMBLE RESSORT

(43) Date of publication of application: 13.12.2017
(73) Proprietor: Covidien LP, Mansfield MA 02048 (US)
(72) Inventor: DING, Weijiang, Shanghai 201114 (CN); LIU, Kai, Longhui, Hunan 722203 (CN)
(74) Representative: Maschio & Soames IP Ltd
(86) International application number: PCT/CN2015/072371
(87) International publication number: WO 2016/123785

(56) References cited:
- EP-A1- 2 392 270
- EP-A2- 2 628 459
- CN-A- 102 178 559
- CN-A- 103 381 106
- CN-A- 104 224 262
- US-A1- 2013 190 753
- US-A1- 2014 025 053
- US-B2- 8 211 119

## Description

### TECHNICAL FIELD

The present disclosure relates to surgical devices for controlling jaw forces, and, more particularly, to surgical devices for controlling jaw forces during a tissue sealing process.

### BACKGROUND

Energy-based medical devices are typically used in conjunction with energy sources (external energy sources or portable energy sources incorporated into the instruments themselves) to apply and control the application of energy to tissue to thermally treat, e.g., heat, tissue to achieve a desired tissue effect. Energy-based surgical forceps, for example, utilize both the mechanical clamping action of the jaw members and the energy provided by the energy source and delivered to tissue to heat tissue grasped between the jaw members to achieve a desired tissue effect, e.g., to seal tissue.

Once tissue is grasped between the jaw members, sealing tissue typically involves heating the grasped tissue during a gassing and drying interval followed by a collagen denaturation and fusion interval. While high jaw forces exerted on tissue facilitate the effectiveness of the gassing and drying interval of the tissue sealing process, high jaw forces during the denaturation and fusion interval of the tissue sealing process tend to promote tissue sticking to the jaw members.

US 2013/190753 A1 describes a forceps with a pair of jaw members, a drive tube and a spring assembly.

US 2014/025053 A1 describes a forceps with a pair of jaw members, a drive tube and a spring assembly with a slip block.

### SUMMARY

Accordingly, the new devices disclosed herein enable a clinician to reduce "tissue stick" through controlling jaw forces during the tissue sealing process. In particular, the presently disclosed devices improve tissue sealing quality with a spring assembly by enabling higher jaw forces during the gassing and drying interval of the tissue sealing process and lower jaw forces during the denaturation and fusion interval of the tissue sealing process.

The invention is defined by the appended independent claim. Preferred embodiments are illustrated in the dependent claims.

Other aspects, features, and advantages will be apparent from the description, the drawings, and the claims that follow.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
Fig. 1 is a perspective view of a forceps according to the principles of the present disclosure;
Fig. 2A is a side, elevational view of the forceps of FIG. 1 with a side portion of a handle thereof removed for clarity, the forceps shown in a first state;
Fig. 2B is an enlarged view of the indicated area of detail shown in Fig. 2A with a shaft assembly of the forceps removed for clarity;
Fig. 2C is an enlarged view of the indicated area of detail shown in Fig. 2A;
Fig. 3A is a side, elevational view of the forceps of FIG. 1 with the side portion of the handle thereof removed for clarity, the forceps shown in a second state;
Fig. 3B is an enlarged view of the indicated area of detail shown in Fig. 3A;
Fig. 3C is an enlarged view of the indicated area of detail shown in Fig. 3A with the shaft assembly of the forceps removed for clarity;
Fig. 4A is a side, elevational view of the forceps of FIG. 1 with the side portion of the handle thereof removed for clarity, the forceps shown in a third state;
Fig. 4B is an enlarged view of the indicated area of detail shown in Fig. 4A;
Fig. 4C is an enlarged view of the indicated area of detail shown in Fig. 4A with the shaft assembly of the forceps removed for clarity; and
Fig. 5 is a schematic illustration of a medical work station and operating console in accordance with the present disclosure.

### DETAILED DESCRIPTION

Particular embodiments of the present disclosure are described hereinbelow with reference to the accompanying drawings in which like reference numerals designate identical or corresponding elements in each of the several views. As used herein, the term "distal" refers to that portion of the system, device, and/or component(s) thereof, that are farther from the user, while the term "proximal" refers to that portion of the system, device, and/or component(s) thereof, that are closer to the user. In the following description, well-known functions or constructions are not described in detail to avoid obscuring the present disclosure in unnecessary detail.

Surgical systems in accordance with the present disclosure can include endoscopic and/or open surgical instruments such as forceps devices, ultrasonic dissection devices, and/or any other suitable surgical devices. Obviously, different electrical and mechanical connections and considerations apply to each particular type of device; however, the aspects and features of the present disclosure remain generally consistent regardless of the particular device used. For a detailed discussion of the construction and operation of example surgical devices, reference may be made to U.S. Patent Application Publication No. 2013/0255063 and/or U.S. Patent No. 8,444,664.

In the interest of brevity, surgical systems of the present disclosure will only be described herein in connection with an endoscopic forceps.

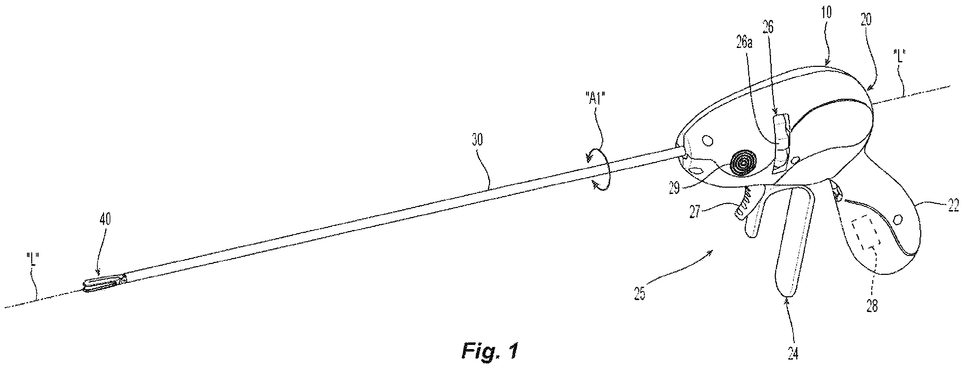

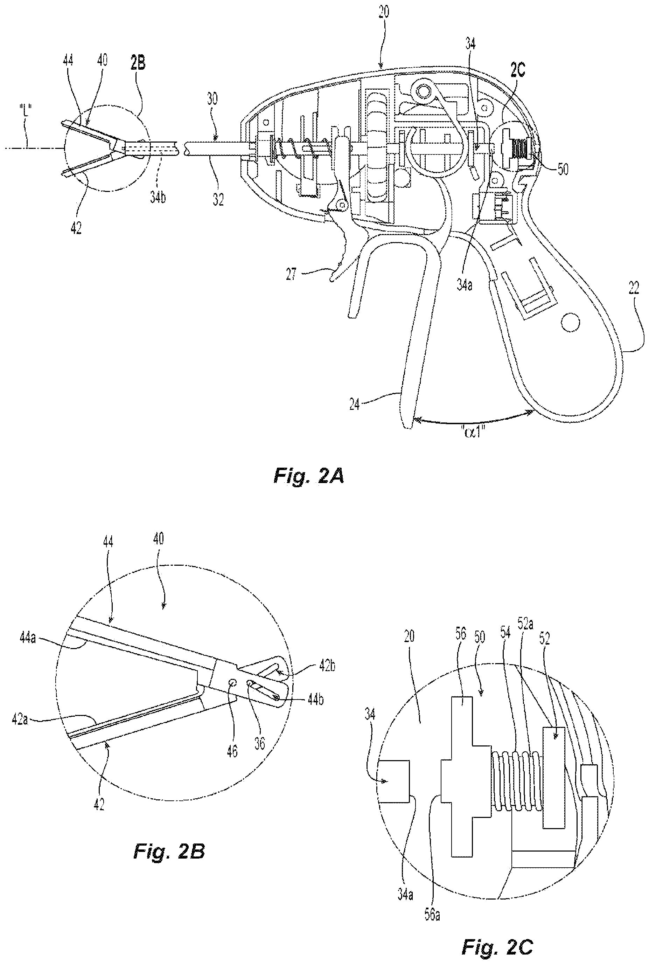

Turning now to Fig. 1, an endoscopic forceps 10 defines a longitudinal axis "L-L" and includes a housing 20, a handle assembly 25, a shaft assembly 30, and an end effector assembly 40. Handle assembly 25 includes a stationary handle 22 integrally formed with housing 20 and a movable handle 24 movable relative to stationary handle 22 to move end effector assembly 40 as described in greater detail below. A rotation assembly 26 is coupled to housing 20 and includes a rotatable knob 26a. Rotatable knob 26a is coupled to shaft assembly 30 (see Fig. 2A) to impart rotation on shaft assembly 30 as illustrated by arrow "A1." A trigger 27 is coupled to housing 20 and a knife assembly (not shown). For a detailed discussion of the construction and operation of an example knife assembly, reference can be made to U.S. Patent Application Publication No. 2013/0138102. Trigger 27 is pivotable relative to housing 20 for selectively translating a knife blade (not shown) through a knife channel (not shown) defined within one or both of first and second jaw members 42, 44 of end effector 40 to cut tissue disposed between first and second jaw members 42, 44.

Housing 20 may support a battery-powered device 28 that is electrically coupled to an activation switch 29 and/or tissue-contacting surfaces 42a, 44a (see FIG. 2B) of end effector assembly 40, for example, to energize tissue-contacting surfaces 42a, 44a of end effector assembly 40 and seal tissue. Alternatively or additionally, forceps 10 includes a cable (not shown) that connects to an energy source (not shown) such as a generator to provide energy to end effector assembly 40 for sealing tissue. In some embodiments, battery-powered device 28 is operatively coupled to trigger 27 and/or movable handle 24 for activating battery-powered device 28 as trigger 27 and/or movable handle 24 are actuated, for example, to a predetermined location/angle relative to stationary handle 22.

With reference to Fig. 2A, shaft assembly 30 includes an outer shaft 32 and a drive tube 34. Drive tube 34 is axially movable along the longitudinal axis "L-L" and relative to outer shaft 32. A proximal end 34a of drive tube 34 is free and a distal end 34b of drive tube 34 is coupled to a proximal end of end effector assembly 40 via a drive pin 36 (see Fig. 2B).

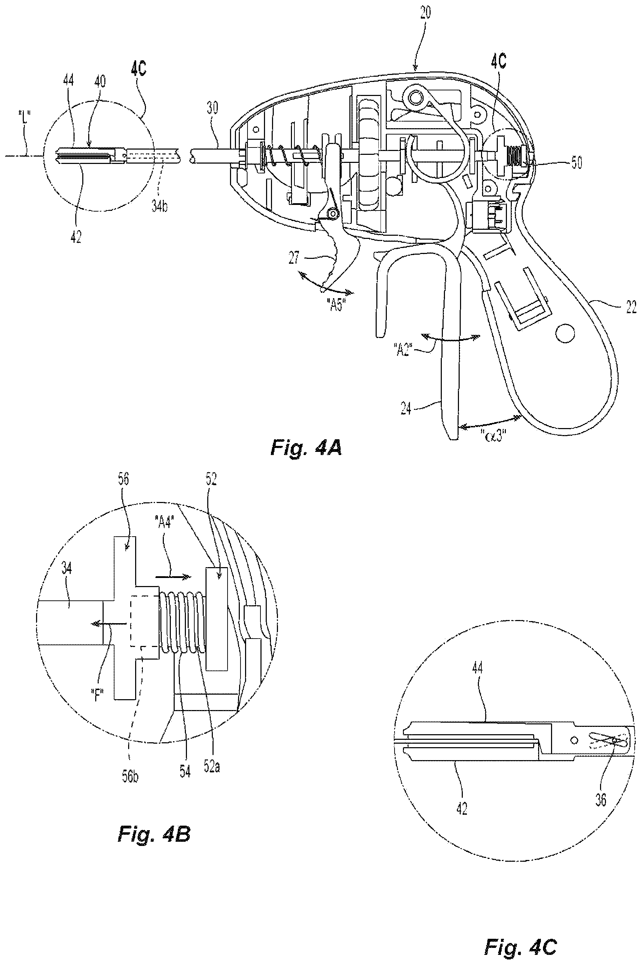

As seen in Fig. 2B, first and second jaw members 42, 44 of end effector 40 are pivotally coupled together by a pivot pin 46. First jaw member 42 includes a tissue contacting surface 42a at a distal end thereof and defines an elongated cam slot 42b through a proximal end thereof. Similarly, second jaw member 44 includes a tissue contacting surface 44a at a distal end thereof and defines an elongated cam slot 44b through a proximal end thereof. Elongated cam slots 42b, 44b have linear profiles that extend along, and transverse to, longitudinal axis "L-L." In certain embodiments, the linear profiles of elongated cam slots 42b, 44b have an elliptical configuration. Elongated cam slots 42b, 44b may include any suitable shape and/or dimension, for example, elongated cam slots 42b, 44b may have an arcuate and/or J-shaped/cane profile, portions of which may be configured to facilitate high and/or low jaw forces between first and second jaw members 42, 44 in response to relative movement of first and/or second jaw members 42, 44. Elongated cam slots 42b, 44b are configured to slidably receive drive pin 36 to enable relative pivoting movement of first and/or second jaw members 42, 44 for positioning first and second jaw members 42, 44 between spaced-apart (Fig. 2B) and approximated states (Fig. 4C) as drive pin 36 slides through elongated cam slots 42b, 44b relative to pivot pin 46.

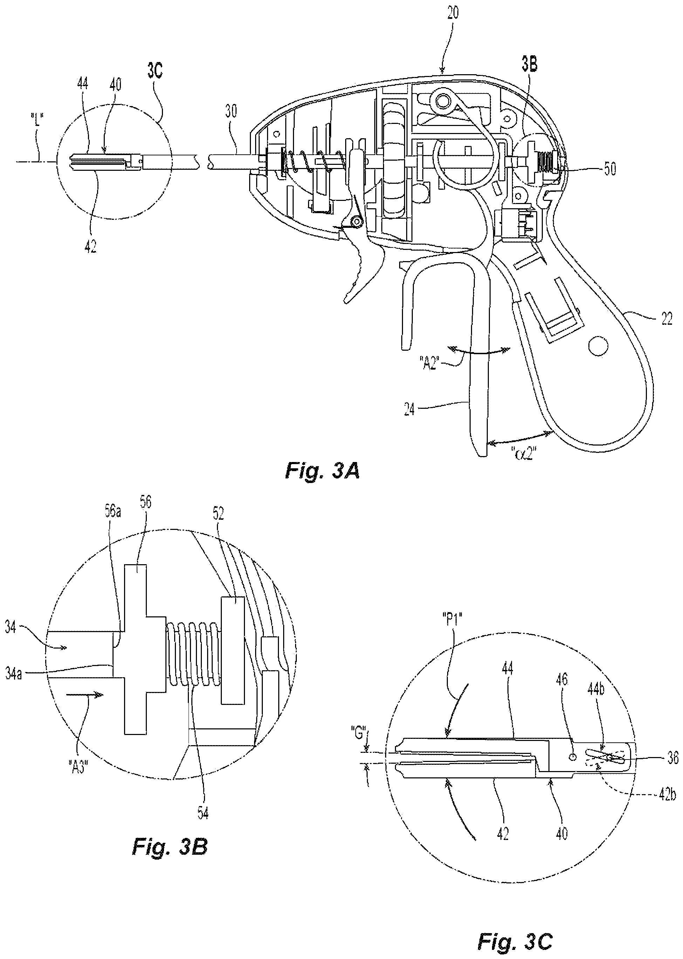

With reference to Figs. 2A and 2C, housing 20 supports a plurality of internal working components including a spring assembly 50. Spring assembly 50 includes a mounting member 52 coupled to housing 20, a spring 54 coupled to mounting member 52, and slip block 56 coupled to spring 54. Mounting member 52 includes a shaft 52a extending distally to slip block 56. Spring 54 is positioned around shaft 52a so that shaft 52a can support spring 54 and enable spring 54 to move axially therealong with minimal splay. Spring 54 may be a compression spring. Slip block 56 extends distally to a distal end 56a configured to engage proximal end 34a of drive tube 34 as seen in Figs 3A and 3B. A bore 56b (Fig. 4B) is defined in slip block 56 that is dimensioned to receive shaft 52a of mounting member 52.

In operation, with reference to Figs. 2A-4C, movable handle 24, which is initially disposed at first angle "α1" relative to stationary handle 22, is actuated/pivoted to approximate first and second jaw members 42, 44 for grasping tissue therebetween. According to the invention, pivoting movement of movable handle 24 toward stationary handle 22 as indicated by arrow "A2" drives drive tube 34 in a proximal direction (illustrated by arrow "A3") from a first position (Fig. 2C), spaced from spring assembly 50, to a second position (Fig. 3B), in which proximal end 34a of drive tube 34 contacts distal end 56a of slip block 56. As drive tube 34 translates axially along longitudinal axis "L-L" in the proximal direction, drive pin 36 slides proximally through elongated cam slots 42b, 44b causing first and second jaw members 42, 44 to pivot toward each other (or one of first and second jaw members 42, 44 toward the other) about pivot pin 46 as illustrated by arrows "P1." Advantageously, a clinician can apply higher jaw forces between first and second jaw members 42, 44 to grasp tissue therebetween with limited effort/resistance from components of forceps 10. When proximal end 34a of drive tube 34 contacts distal end 56a of slip block 56 in this, a second or intermediate state, a gap "G," which may be V-shaped, is formed between first and second jaw members 42, 44. In the intermediate state, movable handle 24 is disposed at a second angle "α2" relative to stationary handle 22. Second angle "α2" may be smaller than first angle "α1."

Once contact is established between proximal end 34a of drive tube 34 and distal end 56a of slip block 56, spring forces of spring 54, which act in the direction "F," counteract movement/compression thereof in the proximal direction, indicated by arrow "A4," thereby dampening or otherwise limiting jaw forces between first and second jaw members 42, 44 by slowing/stopping proximal movement of drive pin 36 and pivoting movement of first and/or second jaw members 42, 44 toward an approximated position (Fig. 4C). The spring forces increase exponentially as spring 54 is compressed further, advantageously controlling the jaw forces between first and second jaw members 42, 44 as first and second jaw members 42, 44 further approximate one another. As slip block 56 compresses spring 56, shaft 52a of mounting member 52 is received within bore 56b to enable slip block 56 to move towards mounting member 52.

Institution of these spring forces in the direction "F" can provide a tactile indicator to a clinician that a predetermined jaw force is established on tissue grasped therebetween. In some embodiments, forceps 10 can include other mechanical and/or electrical components that additionally and/or alternatively provide audible or visual indicators by virtue of a controller (not shown). The predetermined jaw force may also indicate that electrosurgical energy can be activated for initiating tissue sealing. If desired, a clinician can depress activation switch 29 to electrically activate tissue-contacting surfaces 42a, 44a for initiating the tissue sealing process. Advantageously, with limited jaw forces acting between the first and second jaw members 42, 44 against the compression of spring 54, the denaturation and fusion interval of the tissue sealing process can be more effectively controlled as compared to a denaturation and fusion interval with undampened forces that would be higher resulting in tissue sticking, thus improving the quality of the tissue seal by reducing this risk.

In one example use, tissue may be grasped between the first and second jaw members 42, 44 to begin a tissue sealing process. Then, with movable handle 24 actuated a predetermined amount to apply high jaw forces between the first and second jaw members 42, 44, activation switch 29 is actuated to heat, e.g., gasify and dry, the grasped tissue with tissue contacting surfaces 42a, 44a that have been activated by actuation of activation switch 29. Then, with movable handle 24 limited by the spring forces "F," jaw forces between the first and second jaw members 42, 44 are lowered such that the risk of tissue sticking is lowered as the collagen of the grasped tissue denatures and fuses.

Once tissue is sealed, the clinician can actuate trigger 27, as illustrated by arrow "A5," to actuate the knife assembly for selectively cutting the sealed tissue with the knife (not shown) as the knife translates distally through first and/or second jaw members 42, 44. Trigger 27 and movable handle 24 can then be released to, in turn, release tissue. This process can be repeated as desired and/or forceps 10 can be removed from tissue site.

The various embodiments disclosed herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

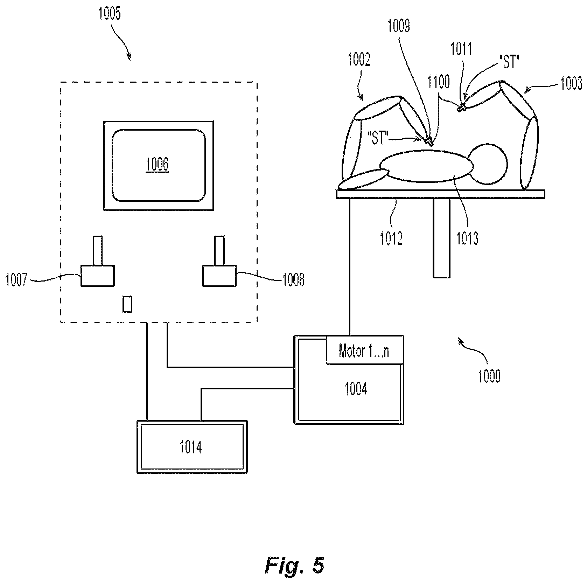

Referring also to Fig. 5, a medical work station is shown generally as work station 1000 and generally may include a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a person (not shown), for example a surgeon, may be able to telemanipulate robot arms 1002, 1003 in a first operating mode.

Each of the robot arms 1002, 1003 may include a plurality of members, which are connected through joints, and an attaching device 1009, 1011, to which may be attached, for example, a surgical tool "ST" supporting an end effector 1100, in accordance with any one of several embodiments disclosed herein, as will be described in greater detail below.

Robot arms 1002, 1003 may be driven by electric drives (not shown) that are connected to control device 1004. Control device 1004 (e.g., a computer) may be set up to activate the drives, in particular by means of a computer program, in such a way that robot arms 1002, 1003, their attaching devices 1009, 1011 and thus the surgical tool (including end effector 1100) execute a desired movement according to a movement defined by means of manual input devices 1007, 1008. Control device 1004 may also be set up in such a way that it regulates the movement of robot arms 1002, 1003 and/or of the drives.

Medical work station 1000 may be configured for use on a patient 1013 lying on a patient table 1012 to be treated in a minimally invasive manner by means of end effector 1100. Medical work station 1000 may also include more than two robot arms 1002, 1003, the additional robot arms likewise being connected to control device 1004 and being telemanipulatable by means of operating console 1005. A medical instrument or surgical tool (including an end effector 1100) may also be attached to the additional robot arm. Medical work station 1000 may include a database 1014, in particular coupled to with control device 1004, in which are stored, for example, pre-operative data from patient/living being 1013 and/or anatomical atlases.

Persons skilled in the art will understand that the structures specifically described herein and shown in the accompanying figures are non-limiting exemplary embodiments, and that the description, disclosure, and figures should be construed merely as exemplary of particular embodiments. Accordingly, the subject matter of the present disclosure is not limited by what has been particularly shown and described.

## Claims

1. A forceps (10), comprising:
a pair of jaw members (42,44);
a drive tube (34) coupled to the pair of jaw members (42,44) and movable to pivot at least a first one of the pair of jaw members relative to a second one of the pair of jaw members between spaced-apart and approximated positions; and
a spring assembly (50) positioned proximal to the drive tube (34), the spring assembly configured to engage the drive tube and limit movement of the drive tube to control relative movement between the pair of jaw members 42,44, wherein the spring assembly (50) includes a slip block (56) and a compression spring (54),
**characterised in that**
the compression spring (34) is configured to bias the slip block toward the drive tube (34), and wherein the drive tube is driven in a proximal direction from a first position spaced from spring assembly (50), to a second position in which
a proximal end (34a) of the drive tube is configured to engage a distal end (56a) of the slip block to decrease closing jaw forces between the pair of jaw members (42,44) while increasing spring forces exerted by the compression spring (54) onto the drive tube as the drive tube moves.

2. The forceps according to claim 1, wherein the pair of jaw members (42,44) each include an electrically-conductive tissue-contacting surface (42a,44a) configured to connect to a source of energy to treat tissue grasped between the pair of jaw members.

3. The forceps according to claim 2, wherein the electrically-conductive tissue-contacting surfaces (42a,44a) are energizable with the source of energy to treat tissue while the slip block (56) is engaged with the drive tube (34).

4. The forceps according to any preceding claim, further including a drive pin (36) coupled to distal end portion (34b) of the drive tube (34), wherein at least one of the pair of jaw members (42,44) defines an elongated cam slot (42b,44b) therein, the drive pin (36) slidably supported within the elongated cam slot (42b,44b) to pivot at least the first one of the pair of jaw members relative the second one of the pair of jaw members.

5. The forceps according to claim 4, wherein the elongated cam slot (42b,44b) has a linear profile along a longitudinal axis defined by the drive tube (34).

6. The forceps according to claim 5, wherein the linear profile has an elliptical configuration.

7. The forceps according to any preceding claim, further including a housing (20) supporting the drive tube (34) and the spring assembly (50).

8. The forceps according to claim 7, further including a handle (24) coupled to the housing (20) and operable to translate the drive tube (34) into engagement with the spring assembly (50).

9. The forceps according to any of claims 1 to 6, further comprising:
a housing (20);
a handle (24) pivotally coupled to the housing;
a shaft assembly (30) defining a longitudinal axis and extending distally along the longitudinal axis from the housing (20) and operatively coupled to the handle (24), wherein the shaft assembly includes the drive tube (34) and the spring assembly (50) is positioned proximally of the shaft assembly; and
an end effector assembly (40) coupled to a distal end portion of the shaft assembly (30), wherein the end effector assembly includes the pair of jaw members (42,44).

10. The forceps according to claim 9, wherein the spring assembly (50) is supported within the housing (20).

## Patentansprüche

1. Zange (10), die Folgendes umfasst:
ein Paar Backenelemente (42, 44);
ein Antriebsrohr (34), das mit dem Paar Backenelemente (42, 44) gekoppelt und bewegbar ist, um wenigstens ein erstes eines des Paars Backenelemente relativ zu einem zweiten einen des Paars Backenelemente zwischen beabstandeten und angenäherten Positionen zu schwenken; und
eine Federanordnung (50), die zu dem Antriebsrohr (34) proximal positioniert ist, wobei die Federanordnung konfiguriert ist, um das Antriebsrohr in Eingriff zu nehmen und eine Bewegung des Antriebsrohrs zu begrenzen, um eine relative Bewegung zwischen dem Paar Backenelemente (42, 44) zu steuern,
wobei die Federanordnung (50) einen Gleitblock (56) und eine Druckfeder (54) beinhaltet, **dadurch gekennzeichnet, dass** die Druckfeder (34) konfiguriert ist, um den Gleitblock in Richtung des Antriebsrohrs (34) vorzuspannen, und wobei das Antriebsrohr in einer proximalen Richtung von einer ersten Position, die von der Federanordnung (50) beabstandet ist, in eine zweite Position angetrieben wird, in der ein proximales Ende (34a) des Antriebsrohrs konfiguriert ist, um ein distales Ende (56a) des Gleitblocks in Eingriff zu nehmen, um Schließbackenkräfte zwischen dem Paar Backenelemente (42, 44) zu verringern, während Federkräfte, die durch die Druckfeder (54) auf das Antriebsrohr ausgeübt werden während sich das Antriebsrohr bewegt, erhöht werden.

2. Zange nach Anspruch 1, wobei das Paar Backenelemente (42, 44) jeweils eine elektrisch leitfähige gewebeberührende Oberfläche (42a, 44a) beinhaltet, die konfiguriert ist, um sich mit einer Energiequelle zu verbinden, um Gewebe zu behandeln, das zwischen dem Paar Backenelemente erfasst wird.

3. Zange nach Anspruch 2, wobei die elektrisch leitfähigen gewebeberührenden Oberflächen (42a, 44a) mit der Energiequelle erregbar sind, um Gewebe zu behandeln, während der Gleitblock (56) das Antriebsrohr (34) in Eingriff nimmt.

4. Zange nach einem der vorhergehenden Ansprüche, die ferner einen Antriebsstift (36) beinhaltet, der mit dem distalen Endabschnitt (34b) des Antriebsrohrs (34) gekoppelt ist, wobei wenigstens eines des Paars Backenelemente (42, 44) einen länglichen Nockenschlitz (42b, 44b) darin definiert, wobei der Antriebsstift (36) innerhalb des länglichen Nockenschlitzes (42b, 44b) verschiebbar gestützt ist, um wenigstens das erste eine des Paars Backenelemente relativ zu dem zweiten einen des Paars Backenelemente zu schwenken.

5. Zange nach Anspruch 4, wobei der längliche Nockenschlitz (42b, 44b) ein lineares Profil entlang einer Längsachse aufweist, die durch das Antriebsrohr (34) definiert ist.

6. Zange nach Anspruch 5, wobei das lineare Profil eine elliptische Konfiguration aufweist.

7. Zange nach einem der vorhergehenden Ansprüche, die ferner ein Gehäuse (20) beinhaltet, das das Antriebsrohr (34) und die Federanordnung (50) stützt.

8. Zange nach Anspruch 7, die ferner einen Griff (24) beinhaltet, der mit dem Gehäuse (20) gekoppelt und betriebsfähig ist, um das Antriebsrohr (34) in Eingriff mit der Federanordnung (50) zu verschieben.

9. Zange nach einem der Ansprüche 1 bis 6, die ferner Folgendes umfasst:
ein Gehäuse (20);
einen Griff (24), der mit dem Gehäuse schwenkbar gekoppelt ist;
eine Schaftanordnung (30), die eine Längsachse definiert und sich entlang der Längsachse von dem Gehäuse (20) distal erstreckt und mit dem Griff (24) wirkgekoppelt ist, wobei die Schaftanordnung das Antriebsrohr (34) beinhaltet und die Federanordnung (50) proximal der Schaftanordnung positioniert ist; und
eine Endeffektoranordnung (40), die mit einem distalen Endabschnitt der Schaftanordnung (30) gekoppelt ist, wobei die Endeffektoranordnung das Paar Backenelemente (42, 44) beinhaltet.

10. Zange nach Anspruch 9, wobei die Federanordnung (50) innerhalb des Gehäuses (20) gestützt ist.

## Revendications

1. Pince (10), comprenant :
une paire d'éléments de mâchoire (42, 44) ;
un tube d'entraînement (34) accouplé à la paire d'éléments de mâchoire (42, 44) et mobile pour faire pivoter au moins un premier élément de la paire d'éléments de mâchoire par rapport à un second élément de la paire d'éléments de mâchoire entre une position espacée et une position rapprochée ; et
un ensemble ressort (50) positionné à proximité du tube d'entraînement (34), l'ensemble ressort étant conçu pour entrer en prise avec le tube d'entraînement et limiter le mouvement du tube d'entraînement pour commander le mouvement relatif entre la paire d'éléments de mâchoire (42, 44),
dans lequel l'ensemble ressort (50) comporte un bloc de glissement (56) et un ressort de compression (54), **caractérisé en ce que** le ressort de compression (34) est conçu pour solliciter le bloc de glissement vers le tube d'entraînement (34), et dans lequel l'entraînement le tube est entraîné dans une direction proximale à partir d'une première position espacée de l'ensemble ressort (50), à une seconde position dans laquelle
une extrémité proximale (34a) du tube d'entraînement est conçue pour entrer en prise avec une extrémité distale (56a) du bloc de glissement afin de diminuer les forces de fermeture de la mâchoire entre la paire d'éléments de mâchoire (42, 44) tout en augmentant les forces de ressort exercées par le ressort de compression (54) sur le tube d'entraînement lorsque le tube d'entraînement se déplace.

2. Pince selon la revendication 1, dans laquelle chaque élément de la paire d'éléments de mâchoire (42, 44) comporte une surface électriquement conductrice en contact avec le tissu (42a, 44a) configurée pour se connecter à une source d'énergie pour traiter le tissu saisi entre la paire d'éléments de mâchoires.

3. Pince selon la revendication 2, dans laquelle les surfaces électriquement conductrices en contact avec le tissu (42a, 44a) peuvent être excitées avec la source d'énergie pour traiter le tissu tandis que le bloc de glissement (56) est en prise avec le tube d'entraînement (34).

4. Pince selon l'une quelconque des revendications précédentes, comportant en outre une broche d'entraînement (36) accouplée à la partie d'extrémité distale (34b) du tube d'entraînement (34), au moins l'un des éléments la paire d'éléments de mâchoire (42, 44) définissant une fente de came allongée (42b, 44b) à l'intérieur, la broche d'entraînement (36) étant supportée de manière coulissante à l'intérieur de la fente de came allongée (42b, 44b) pour faire pivoter au moins le premier élément de la paire d'éléments de mâchoire par rapport au second élément de la paire d'éléments de mâchoire.

5. Pince selon la revendication 4, dans laquelle la fente de came allongée (42b, 44b) a un profil linéaire le long d'un axe longitudinal défini par le tube d'entraînement (34).

6. Pince selon la revendication 5, dans laquelle le profil linéaire a une configuration elliptique.

7. Pince selon l'une quelconque des revendications précédentes, comportant en outre un boîtier (20) supportant le tube d'entraînement (34) et l'ensemble ressort (50).

8. Pince selon la revendication 7, comportant en outre une poignée (24) accouplée au boîtier (20) et pouvant être actionnée pour translater le tube d'entraînement (34) en prise avec l'ensemble ressort (50).

9. Procédé selon l'une quelconque des revendications 1 à 6, comprenant en outre :
un boîtier (20) ;
une poignée (24) accouplée de manière pivotante au boîtier ;
un ensemble arbre (30) définissant un axe longitudinal et s'étendant de manière distale le long de l'axe longitudinal à partir du boîtier (20) et accouplé de manière fonctionnelle à la poignée (24), l'ensemble arbre comportant le tube d'entraînement (34) et l'ensemble ressort (50) étant positionné à proximité de l'ensemble arbre ; et
un ensemble effecteur d'extrémité (40) accouplé à une partie d'extrémité distale de l'ensemble arbre (30), l'ensemble effecteur d'extrémité comportant la paire d'éléments de mâchoire (42, 44).

10. Pince selon la revendication 9, dans laquelle l'ensemble ressort (50) est supporté à l'intérieur du boîtier (20).
